# EUROPEAN PATENT APPLICATION

(11) **EP 2 216 047 A1**
(43) Date of publication of application: **11.08.2010**
(21) Application number: 08841616.9
(22) Date of filing: 23.10.2008
(51) Int. Cl.: A61K 45/00, A61K 31/336, A61P 1/04, A61P 11/06, A61P 13/12, A61P 17/06, A61P 19/00, A61P 25/00, A61P 35/00, A61P 37/00, A61P 37/02, A61P 37/06, A61P 37/08, A61P 43/00, C07D 303/48

(54) **REGULATOR FOR SIGNALING OF TOLL-LIKE RECEPTOR, WHICH COMPRISES CATHEPSIN INHIBITOR AS ACTIVE INGREDIENT**

(30) Priority: 24.10.2007 JP 2007276937
(71) Applicant: National University Corporation Tokyo Medical and Dental University, Bunkyo-ku Tokyo 113-8510 (JP); Nippon Chemiphar Co., Ltd., Chiyoda-ku Tokyo 1010032 (JP)
(72) Inventor: TAKAYANAGI, Hiroshi, Tokyo 150-0047 (JP); ASAGIRI, Masataka, La Jolla, California 9209-30375 (US); HIRAI, Toshitake, Noda-shi Chiba 278-0036 (JP)
(74) Representative: Hiebl, Inge Elisabeth
(86) International application number: PCT/JP2008/069235
(87) International publication number: WO 2009/054454

(57) **Abstract**

[Problems] To provide a modulator for signaling of TLR

[Means for solving the problems] A medicament containing cathepsin inhibitor such as monosodium (2S,3S)-3-[[(1S)-1-isobutoxymethyl-3-methylbutyl]carbamoyl]oxirane-2-carboxylate as a active ingredient is used as a modulator for signaling of TLR, a therapeutic agent for treating diseases associated with TLR signaling, a therapeutic agent for treating diseases associated,with induction of Th17 cells, a therapeutic agent for treating diseases associated with production of IL-6, IL-12, IL-17, or IL-23, or a therapeutic agent for treating systemic lupus erythematosus, lupus nephritis, crohn's disease, psoriasis, or acute disseminated encephalomyelitis.

## Description

### Field of the invention

The present invention relates to a modulator (regulator) for signaling of TLR (Toll-like receptor), which contains a cathepsin inhibitor as an active ingredient.

### Background of the invention

Toll-like receptor (TLR) is a receptor protein located on a cell surface. TLR recognizes pathogen-associated molecular patterns (PAMPs), and participates in signaling to activate natural immunity.

It has been estimated that humans have ten subtypes of TLR. TLR1, TLR2, TLR4, and TLR6 localized on a cell surface recognize a glycoprotein or a lipid component derived from microbes. TLR3, TLR7, TLR8, and TLR9 localized on an intracellular endosome recognize a nucleic acid derived from microbes.

When TLR recognizes a foreign object, it produces inflammatory cytokine or interferon (IFN). Accordingly, it is important for control on diseases to regulate the function of TLR. For example, Patent document 1 proposes using 4-primary amino-quinoline, which is an antagonist of TLR3, TLR7, TLR8, or TLR 9, for diseases such as autoimmune disease, inflammation, allergosis, or asthma.

CD⁴⁺ T helper cells have so far been known as T helper subset 1 (Th1) cells concerning cellular immunity and Th2 cells concerning humoral immunity.

A new T cell subset has recently been found as Th17, which plays an important role in an allergic response, autoimmunity, and phylaxis for extracellular bacterial growth.

It has also been known that Th17 is maintained and induced with interleukin-23 (IL-23).

It has further been known that Th17 produces IL-17. Patent document 2 proposes using an agonist or an antagonist of Th17 for prophylaxis of tumor.

Patent documents 3, 4, and 5 propose applying an inhibitor of IL-17 activity for prophylaxis of multiple sclerosis or rheumatoid arthritis.

Patent document 6 proposes that treatment with an antagonist of IL-23 (anti IL-23 or anti IL-23 receptor antibody) prevents production of IL-17 to result in prophylaxis of inflammatory diseases (such as rheumatoid arthritis, multiple sclerosis).

Cathepsins constitute a family of papain-like lysosomal cysteine proteases that prefer low pH condition to exert their activity. Although the cathepsins were once recognized as being essentially non-specific scavengers of cellular proteins, they also have certain cell-type-specific functions based on spatiotemporal expression patterns and substrate specificities.

Cathepsin K of the family is a protease particularly expressed in osteoclasts compared with (the?) gene expression in other cells. It has been found that the expression level of cathepsin K in osteoclasts is about 20 to 500 times as high as that of other cysteine proteases. It has been confirmed that cathepsin K is an enzyme whose much expression is specific to osteoclasts. It has been understood that cathepsin K is the most important enzyme (protease) secreted from osteoclasts degradating bone matrix.

Patent document 7 expects monosodium (2S,3S)-3-[[(1S)-1-isobutoxymethyl-3-methylbutyl]carbamoyl]oxirane-2-carboxylate, an agent inhibiting cathepsin K to be an agent for prophylaxis of osteoporosis.

However, it has not yet been known that a cathepsin K inhibitor relates to the TLR signaling. It has also not yet been known that the cathepsin K inhibitor is effective in production of Th17 or prophylaxis of diseases relating to production of IL-6, I1-12, IL-17, or IL-23.
Patent document 1: JP 2007-524615 A
Patent document 2: JP 2006-520781 A
Patent document 3: JP 2007-511593 A
Patent document 4: JP 2000-186046 A
Patent document 5: JP 2004-517918 A
Patent document 6: JP 2006-514004 A
Patent document 7: WO 99/11640 A1

### Disclosure of the invention

### Problems to be solved by the invention

The object of the present invention is to provide a modulator effective in TLR signaling which contains a cathepsin inhibitor as an active ingredient, and to provide an agent for treating diseases involved in TLR signaling.

### Means to solve the problem

The present invention provides a modulator for signaling of TLR which contains a cathepsin inhibitor as an active ingredient.

The invention also relates to a therapeutic agent for treating diseases associated with signaling of TLR which contains a cathepsin inhibitor as an active ingredient.

The invention further relates to a therapeutic agent for treating diseases associated with induction of Th17 cells which contains a cathepsin inhibitor as an active ingredient

The invention further relates a therapeutic agent for treating diseases associated with production of IL-6, IL-12, IL-17, or IL-23 which contains a cathepsin inhibitor as an active ingredient.

The invention further relates to a therapeutic agent for treating systemic lupus erythematosus, lupus nephritis, crohn's disease, psoriasis, or acute disseminated encephalomyelitis which contains a cathepsin inhibitor as an active ingredient.

### The best mode of the invention

In the modulator effective in TLR signaling which contains a cathepsin inhibitor as an active ingredient or in the agent for treating disease relating to TLR signaling which contains a cathepsin inhibitor as an active ingredient, TLR can be selected from the group consisting of TLR3, TLR7, TLR8, and TLR9, and preferably is TLR9.

The disease relating to TLR signaling, the disease involved in induction of Th17 cells, or the disease involved in production of IL-6, IL-12, IL-17, or IL-23 can be selected from the group consisting of immune disease, bone disease, and cancer disease.

The immune diseases include autoimmune disease such as systemic lupus erythematosus, lupus nephritis, crohn's disease, psoriasis, acute disseminated encephalomyelitis, and multiple sclerosis, infectious disease, and asthma, and particularly is autoimmune disease.

The cathepsin inhibitor of the present invention includes cathepsin B inhibitor, cathepsin H inhibitor, cathepsin K inhibitor, cathepsin L inhibitor, and cathepsin S inhibitor, preferably is cathepsin B inhibitor, cathepsin H inhibitor, or cathepsin K inhibitor, and more preferably is cathepsin K inhibitor. The cathepsin inhibitors are described in various Patent documents such as WO96/30354, WO97/21694, WO98/47887, WO99/11640, WO01/049288, WO01/058886, WO01/070232, WO03/053331, WO03/075836, WO03/091202 WO2004/108661, WO2005/000800, WO2005/066180, WO2007/025774, and JP 11(1999)-263783 A. The cathepsin inhibitors are also described in Non-patent documents such as T. Yasuma et al., J. Med. Chem. 1998, 41, 4301-4308; N. Katumuma et al., FEBS Lett., 1999, 458, 6-10; T. Otsuka et al., J. Antibiot., 1999, 52, 536-541; R.W. Marquis et al., J. Med. Chem. 2001, 44, 1380-1395; J. Robichaud et al., J. Med. Chem. 2003, 46, 3709-3727; and C.S. Li et al., Bioorg., Med. Chem. Lett., 2006, 16, 1985-1989.

In the present invention, the cathepsin inhibitor preferably is N-[1-[(cyanomethyl)carbamoyl]cyclohexyl]-4-(4-propylpiperazin-1-yl)benzamide, N-[(S)-3-methyl-1-[[(4S,7R)-7-methyl-3-oxo-1-(pyridin-2-ylsulfonyl)hexahydro-1H-azepin-4-yl]carbamoyl]butyl]-1-benzofuran-2-carboxamide, (2R)-N-cyanomethyl-4-methyl-2-(4'-piperazin-1-yl-1,1'-biphenyl-3-yl)pentanamide, N-[3-[(2Z)-2-(3-methyl-1,3-thiazolidin-2-ylidene)hydrazino]-2,3-dioxo-1-tetrahydro-2H-pyran-4-ylpropyl]cycloheptanecarboxamide, N-cyanomethyl-4-methyl-2-[2,2,2-trifluoro-1-(4'-methylsulfonyl-1,1'-biphenyl-4-yl)ethylamino]pentanamide, or monosodium (2S,3S)-3-[[(1S)-1-isobutoxymethyl-3-methylbutyl]carbamoyl]oxirane-2-carboxylate.

The cathepsin inhibitor of the present invention preferably is E-64d.

The cathepsin inhibitor of the present invention also preferably is an epoxysuccinamide derivative described below.
(1) Epoxysuccinamide derivative represented by the formula (1) or a physiologically acceptable salt thereof: 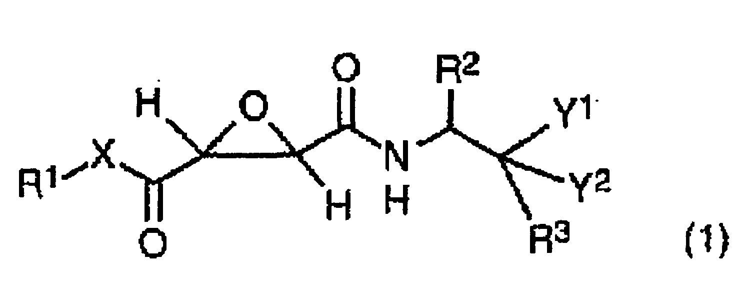 wherein
   R¹ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
   R² represents an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
   R³ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
   X represents -O- or -NR⁴- in which R⁴ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
   Y¹ represents OR⁵ in which R⁵ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, SR⁶ in which R⁶ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, or NR⁷R⁸ in which R⁷ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, and R⁸ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
   Y² represents a hydrogen atom or an alkyl group having 1 to 10 carbon atoms; or
   Y¹ and Y² in combination with each other can form =O, =S, =N-R⁹ in which R⁹ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, or =N-OR¹⁰ in which R¹⁰ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
   provided that each of the alkyl groups for R⁵ to R¹⁰ can have one or more substituents selected from the group consisting of hydroxyl, amino, an alkylamino group having 1 to 6 carbon atoms, a dialkylamino group having 2 to 12 carbon atoms in total, an alkoxy group having 1 to 6 carbon atoms, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, and guanidino; and each of the aryl groups and the heterocyclic groups for R¹ to R¹⁰ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, amidino, and guanidino.
(2) The compound of (1), wherein R¹ is a hydrogen atom or an alkyl group having 1 to 6 carbon atoms.
(3) The compound of (1) or (2), wherein R² is an alkyl group having 1 to 6 carbon atoms, phenyl, or benzyl.
(4) The compound of one selected from (1) to (3), wherein R³ is a hydrogen atom or an aryl group having 6 to 20 carbon atoms.
(5) The compound of one selected from (1) to (4), wherein X is -O-.
(6) The compound of one selected from (1) to (5),
   wherein Y¹ is hydroxyl, an alkoxy group having 1 to 6 carbon atoms, acetoxy, or an aralkyloxy group comprising an aryl group having 6 to 20 carbon atoms and an alkyl group having 1 to 6 carbon atoms.
(7) The compound of one selected from (1), (2), and (5), wherein R² is isobutyl or isopropyl, R³ is a hydrogen atom, Y¹ is OR⁵ in which R⁵ has the meaning defined in (1), and Y² is a hydrogen atom.
(8) The compound of one selected from (1), (2), and (5), wherein R² is isobutyl or isopropyl, R³ is an aryl group having 6 to 20 carbon atoms, Y¹ is OR⁵ in which R⁵ has the meaning defined in (1), and Y² is a hydrogen atom.
(9) The compound of one selected from (1) to (5),
   wherein Y¹ and Y² in combination with each other forms =O.
(10) The compound of one selected from (1) to (9),
   wherein the physiologically acceptable salt is an alkali metal salt.

Examples of the epoxysuccinamide derivatives represented formula (1) are set forth in the following Table 1 (Tables 1-1, 1-2, 1-3, and 1-4).

In Tables 1-1, 1-2, 1-3, and 1-4, R¹, R², R³, X, Y¹, and Y² mean those shown in the aforementioned formula (1). In the following Tables, each symbol means the following: H: hydrogen, Me: methyl, Et: ethyl, Ph: phenyl, Bn: benzyl, iPr: isopropyl, iBu: isobutyl, sBu: sec-butyl, tBu: tert-butyl, cHex: cyclohexyl, 4-MePh: 4-methylphenyl, 4-ClPh: 4-chlorophenyl, 4-tBuPh: 4-tert-butylphenyl, 4'-HOBn: 4'-hydroxybenzyl.

**Table 1-1**

| No. | R¹ | R² | R³ | X | Y¹ | y² |
|---|---|---|---|---|---|---|
| 1 | Et | iBu | Ph | O | OH | H |
| 2 | H | iBu | Ph | O | OH | H |
| 3 | iPr | iBu | Ph | O | OH | H |
| 4 | Et | iBu | Ph | NH | OH | H |
| 5 | Ph | iBu | Ph | NMe | OH | H |
| 6 | Et | iBu | 4-MePh | O | OH | H |
| 7 | Et | iBu | 4-ClPh | O | OH | H |
| 8 | Et | iBu | iPr | O | OH | H |
| 9 | Et | sBu | Ph | O | OH | H |
| 10 | Et | Ph | Ph | O | OH | H |
| 11 | Et | iBu | Ph | O | OMe | H |
| 12 | Et | iBu | Ph | O | OEt | H |
| 13 | Et | iBu | H | O | OH | H |
| 14 | Et | iBu | Me | O | OH | Me |
| 15 | Et | iBu | Ph | O | =O (together with Y²) | |
| 16 | Et | iPr | Ph | O | =O (together with Y²) | |
| 17 | Et | iPr | Ph | O | =S (together with Y²) | |
| 18 | H | sBu | Ph | O | OH | H |
| 19 | H | Ph | Ph | O | OH | H |
| 20 | H | iBu | Ph | O | OMe | H |
| 21 | H | iBu | H | O | OH | H |

**Table 1-2**

| No. | R¹ | R² | R³ | X | Y¹ | Y² |
|---|---|---|---|---|---|---|
| 22 | Et | iBu | Ph | O | acetoxy | H |
| 23 | Et | iBu | Ph | O | benzoyloxy | H |
| 24 | iPr | iBu | Ph | O | OMe | H |
| 25 | Bn | iBu | Ph | O | OMe | H |
| 26 | Et | iBu | Ph | O | benzyloxy | H |
| 27 | Et | iBu | H | O | benzyloxy | H |
| 28 | Et | iBu | H | O | 4'-chlorobenzyloxy | H |
| 29 | Et | iBu | H | O | 2'-methylbenzyloxy | H |
| 30 | Et | iBu | H | O | 3',4',5'-trimethoxybenzyloxy | H |
| 31 | Et | iPr | H | O | 2'-chlorobenzyloxy | H |
| 32 | Et | iPr | H | O | 4'-methylbenzyloxy | H |
| 33 | Et | iBu | H | O | 4'-amidinobenzyloxy | H |
| 34 | Et | iBu | H | O | 4'-guadininobenzyloxy | H |
| 35 | Et | iBu | H | O | carboxymethoxy | H |
| 36 | Et | iBu | H | O | (2-ethoxycarbonylethyl)oxy | H |
| 37 | Et | iBu | H | O | (1-piperadinylcarbonyl)methoxy | H |
| 38 | Et | iBu | H | O | isobutylamino | H |

**Table 1-3**

| No. | R¹ | R² | R³ | | X Y¹ | Y² |
|---|---|---|---|---|---|---|
| 39 | Et | iBu | H | O | diethylamino | H |
| 40 | Et | iBu | H | O | benzylamino | H |
| 41 | Et | iBu | H | O | benzoylamino | H |
| 42 | Et | iBu | H | O | benzylcarbonylamino | H |
| 43 | Et | iBu | H | O | isobutoxy | H |
| 44 | Et | Bn | H | O | benzyloxy | H |
| 45 | Et | Bn | H | O | isobutoxy | H |
| 46 | Et | 4'-HOBn | H | O | benzyloxy | H |
| 47 | Et | iBu | H | O | diphenylmethoxy | H |
| 48 | H | iBn | Ph | O | benzyloxy | H |
| 49 | H | Bn | H | O | benzyloxy | H |
| 50 | H | iBu | H | O | benzyloxy | H |
| 51 | H | iBu | H | O | (4-guanidinobutyl)oxy | H |
| 52 | H | iBu | H | O | (2-ethylaminoethyl)oxy | H |
| 53 | H | iBu | H | O | (2-diethylaminoethyl)oxy | H |

**Table 1-4**

| No. | R¹ | R² | R³ | X | Y¹ | Y² |
|---|---|---|---|---|---|---|
| 54 | Et | iBu | cHex | O | OH | H |
| 55 | Et | iBu | cHex | O | OMe | H |
| 56 | Et | iBu | H | O | 2'-methylbenzyloxy | H |
| 57 | Et | iBu | H | O | 2',6'-dimethylbenzyloxy | H |
| 58 | Et | iBu | H | O | 4'-isopropylbenzyloxy | H |
| 59 | Et | iBu | H | O | 2'-chlorobenzyloxy | H |
| 60 | Et | iBu | H | O | 4'-trifluoromethylbenzyloxy | H |
| 61 | Et | iBu | H | O | 4'-cyanobenzyloxy | H |
| 62 | Et | iBu | H | O | 3'-aminobenzyloxy | H |
| 63 | Et | iBu | H | O | (3-pyridyl)methoxy | H |
| 64 | Et | iBu | H | O | (3-thienyl)methoxy | H |
| 65 | Et | iBu | H | O | (2-benzoimidazolyl)methoxy | H |
| 66 | Et | iBu | H | O | (1-naphthyl)methoxy | H |
| 67 | Et | iBu | H | O | 2-naphthyloxy | H |
| 68 | Et | iBu | H | O | phenoxy | H |
| 69 | Et | iBu | H | O | 2-phenylethoxy | H |
| 70 | Et | iBu | H | O | 3-phenylpropoxy | H |
| 71 | Et | iBu | H | O | ethoxy | H |
| 72 | Et | iBu | H | O | (3-methylbutyl)oxy | H |
| 73 | Et | iBu | H | O | hexyloxy | H |
| 74 | Et | iBu | H | O | cyclopropylmethoxy | H |
| 75 | Et | iBu | H | O | cyclohexylmethoxy | H |
| 76 | Et | iBu | H | O | (2-methyl-2-propenyl)oxy | H |
| 77 | Et | iBu | H | O | (3-methyl-2-butenyl)oxy | H |
| 78 | Et | iBu | H | O | 2-methoxyethoxy | H |
| 79 | Et | iBu | H | O | (dimethylcarbamoyl)methoxy | H |
| 80 | Et | iBu | H | O | 3-(4-benzyl-1-piperadinyl)propoxy | H |
| 81 | Et | iBu | H | O | 4-diethylaminobutyloxy | H |
| 82 | iPr | iBu | H | O | isobutoxy | H |
| 83 | tBu | iBu | H | O | isobutoxy | H |
| 84 | cHex | iBu | H | O | isobutoxy | H |
| 85 | Ph | iBu | H | O | isobutoxy | H |
| 86 | 4-tBuPh | iBu | H | O | isobutoxy | H |
| 87 | H | iBu | H | O | isobutoxy | H |
| 88 | Et | iPr | H | O | isobutoxy | H |
| 89 | Et | sBu | H | O | isobutoxy | H |
| 90 | Et | iPr | H | O | benzyloxy | H |
| 91 | Et | Bn | H | O | benzyloxy | H |
| 92 | Et | iBu | H | O | 2-methylpropionylamino | H |
| 93 | Et | iBu | H | O | hexanoylamino | H |
| 94 | Et | iBu | H | O | N-benzyl-N-methylamino | H |
| 95 | Et | iBu | H | O | N-hexyl-N-methylamino | H |

The epoxysuccinamide derivative represented by the formula (1) can be employed in the form of a physiologically acceptable salt. For example, in the case that R¹ is a hydrogen atom and X is -O-, it forms a salt with an alkali metal (e.g., sodium or potassium), an alkaline earth metal (e.g., calcium), or an organic amine (e.g., triethylamine or pyridine).

Processes for preparing epoxysuccinamide derivatives of the formula (1) are described below. The epoxysuccinamide derivative of the formula (1) can be prepared from the known compound by a process involving production of amide-bonding, esterification, or hydrolysis. The respective reaction schemes are illustrated below.

### 1) Production of amide-bonding (1)

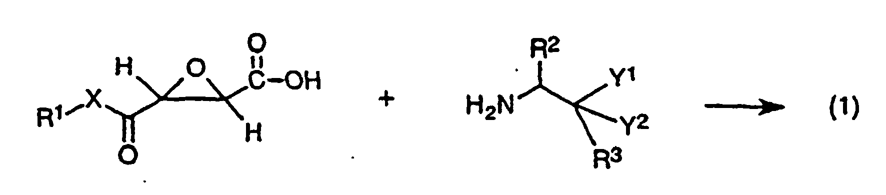

### 2) Production of amide-bonding (2)

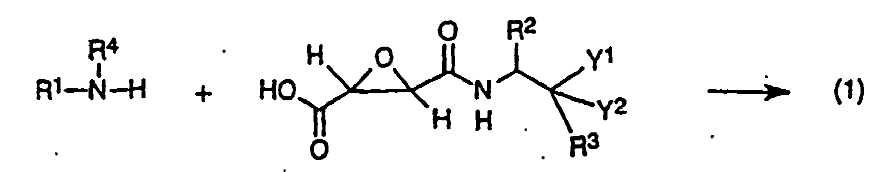

### 3) Esterification

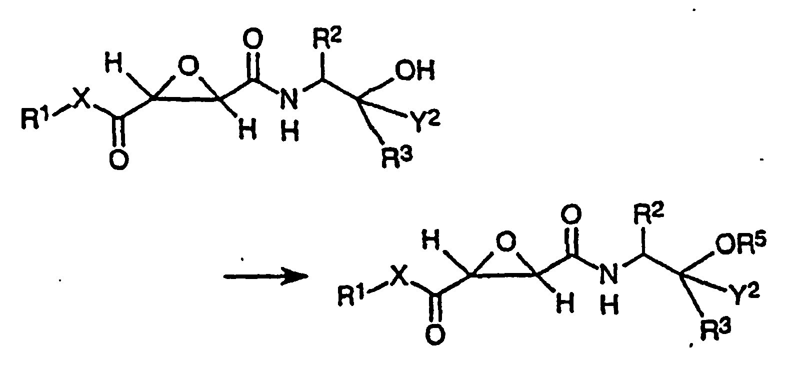

### 4) Esterification

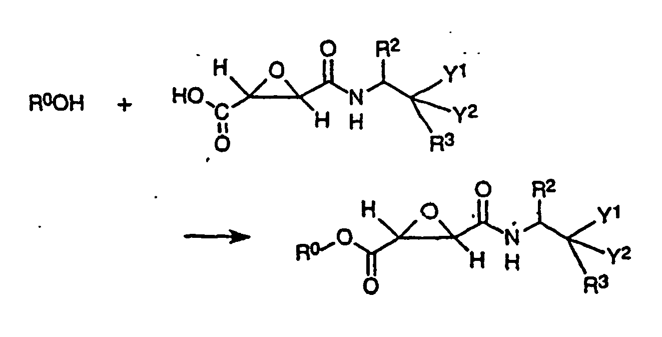

### 5) Hydrolysis

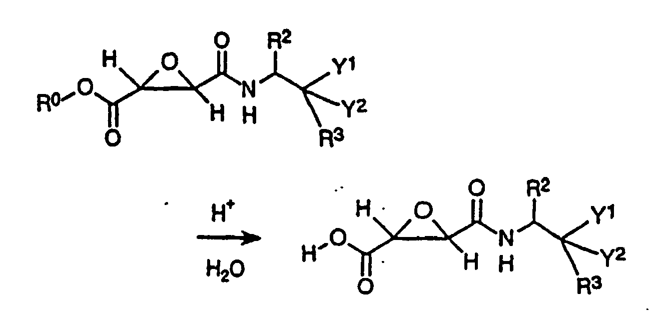

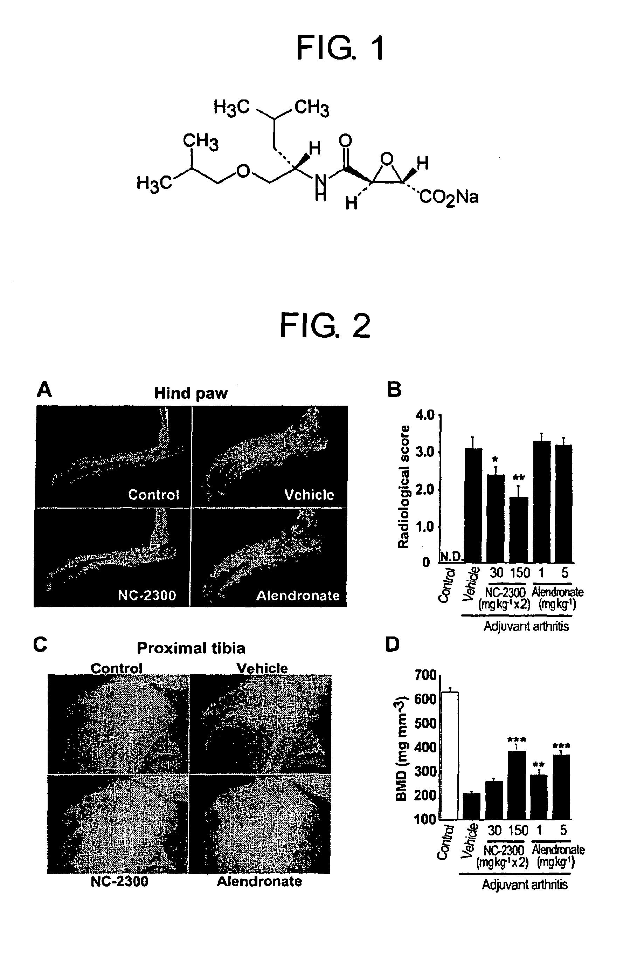

In the present invention, the cathepsin inhibitor preferably is monosodium (2S,3S)-3-[[(1S)-1-isobutoxymethyl-3-methylbutyl]carbamoyl]oxirane-2-carboxylate (shown in Fig. 1), which is hereinafter referred to as NC-2300.

Pharmacological tests are described below.

Rat adjuvant arthritis was treated with oral administration of NC-2300. The effects were compared with those of alendronate, which is one of the bisphosphonates and an inhibitor of osteoclastic bone resorption in clinical use. Bone loss in arthritis occurs mainly in two forms: bone erosion at the inflamed joints and periarticular osteoporosis. Radiological analysis revealed that NC-2300, but not alendronate, markedly suppressed bone erosion in the ankle (Fig. 2, A and B), although bone mineral density (BMD) analysis showed that both compounds had a comparable inhibitory effect on periarticular osteoporosis in the proximal tibia (Fig. 2, C and D). Alendronate did not efficiently suppress bone erosion, partly because osteoclasts which are stimulated with inflammatory cytokines become resistant to bisphosphonates. These results suggest that cathepsin K is a promising target for preventing bone erosion as well as periarticular osteoporosis in arthritis.

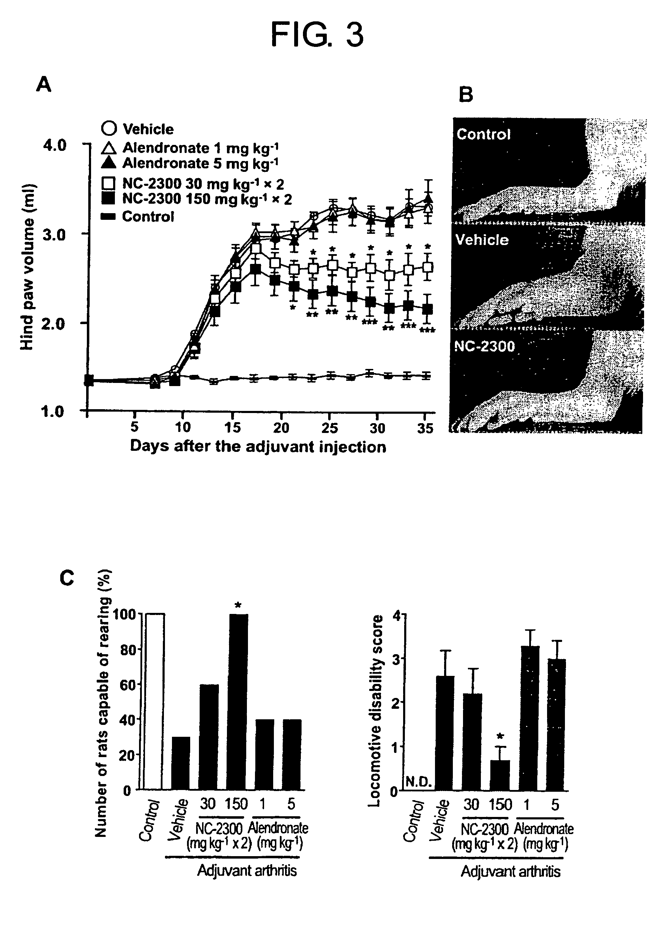

It was found that NC-2300 ameliorated paw swelling in the course of arthritis (Fig. 3A and 3B) and improved locomotive activity (Fig. 3C) without affecting the onset rate of arthritis. NC-2300 ameliorated inflammation even when it was administered after the onset of disease. These results indicate that cathepsin K functions in cells other than osteoclasts and plays a crucial role in autoimmune inflammation.

In adjuvant arthritis, local injection of adjuvant stimulates antigen presentation by dendritic cells, leading to T cell autoimmunity, the production of inflammatory cytokines by macrophages and osteoclast-mediated bone destruction. The adjuvant effects are mainly dependent on the pathogen-associated molecular patterns (PAMPs)-induced activation of TLR signaling. Therefore, the expression of cathepsin K in T cells, macrophages, and dendritic cells were analyzed, and the effects of a cathepsin K inhibitor on the activation of these cells were examined.

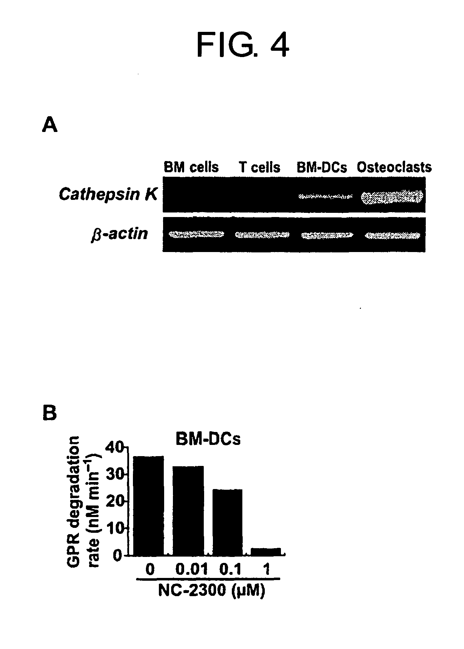

Cathepsin K mRNA was barely detected in non-adherent BM cells or splenic T cells by RT-PCR analysis (Fig. 4A). Consistently, NC-2300 had no effect on the proliferation or IL-2 production of T cells stimulated with anti-CD3/CD28 antibodies. Expression of cathepsin K in macrophages has been reported, but NC-2300 had no significant effects on the activation of BM-derived macrophages stimulated by PAMPs. BM-derived DCs were revealed to express a detectable level of cathepsin K mRNA (Fig. 4A). Although cathepsin K mRNA expression in DCs was much lower than that in osteoclasts, cathepsin K activity in DCs was confirmed using a fluorogenic cathepsin K-specific substrate, Z-Gly-Pro-Arg-MCA (Z, benzyloxycarbonyl; MCA, 4-methylcoumary-7-amide), and the activity was inhibited by NC-2300 in a dose-dependent manner (Fig. 4B).

The role of cathepsin K in antigen presentation by DCs was investigated. As the result, it was considered that cathepsin K activity is not required for the antigen uptake, processing, or presentation by DCs.

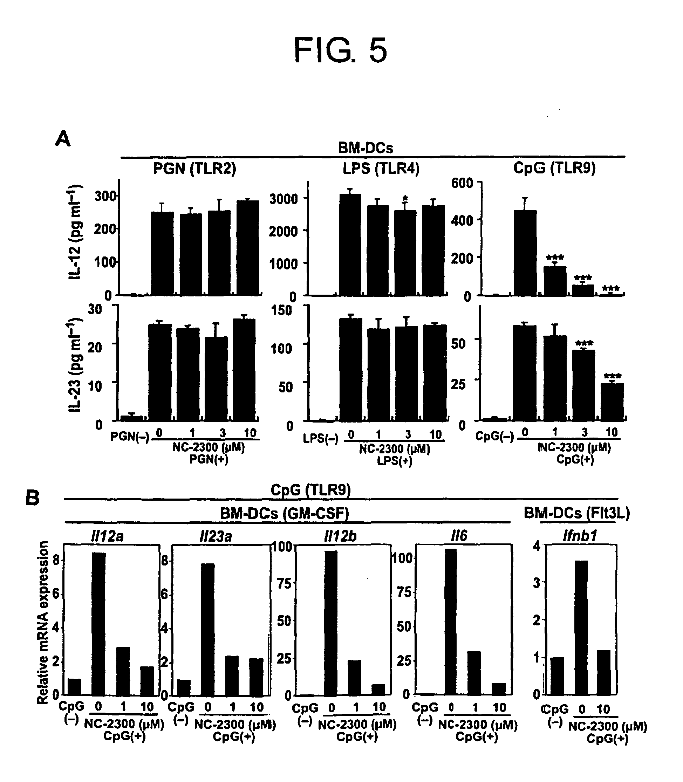

Complete Freund's adjuvant (CFA) used for the induction of adjuvant arthritis is a mixture of incomplete Freund's adjuvant and killed mycobacteria, which contain various PAMPs including TLR2, TLR4 and TLR9 agonists. Therefore, the responses of DCs to these PAMPs were tested with or without cathepsin K inactivation. Enzyme-linked immunosorbent assay (ELISA) revealed that the production of cytokines such as IL-12 and IL-23 by DCs was significantly inhibited by NC-2300 when they were stimulated with oligodeoxynucleotides containing unmethylated CpG motif (CpG: the TLR9 agonist), but not with peptidoglycan (PGN: the TLR2 agonist) or lipopolysaccharide (LPS: the TLR4 agonist) (Fig. 5A). TLR9 mRNA expression was not markedly affected by cathepsin K inactivation. CpG-induced expression of IL-6, IL-12 and IL-23 in BM-DCs was downregulated at the mRNA level (Fig. 5B), and IFN-1 production by Flt3L-induced DCs in response to CpG was suppressed by NC-2300. These results suggest that cathepsin K plays an important role in the gene induction program regulated by TLR9 signaling.

DCs derived from mouse spleen was treated with poly(I:C) (TLR3 ligand) and R837 (TLR7, 8 ligand). The effect of NC-2300 on cytokine mRNA expression of the DCs was measured.

The DCs were prepared from mouse spleen. The total RNA was extracted 3 hours after stimulation.

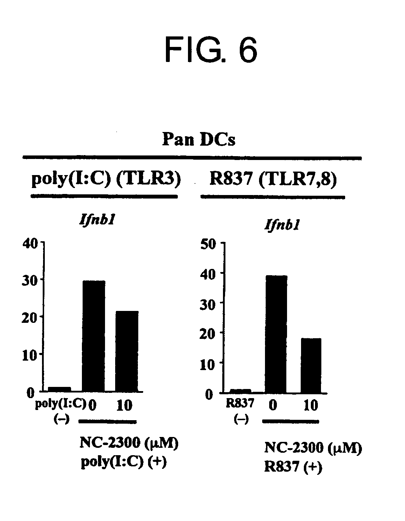

As the result, expression of IFN-1 in the DCs derived from mouse spleen before the treatment with poly(I:C) and R837 was downregulated by NC-2300 at the mRNA level (Fig. 6).

Further, E64d, a cathepsin inhibitor also downregulated expression of IFN-1 at the mRNA level in the DCs derived from mouse spleen.

It has been reported that DNase treatment of the mycobacterial components in CFA greatly reduces the severity of adjuvant arthritis without affecting the induction efficiency. Supplementation of DNase-treated mycobacterial components with CpG DNA recovers the ability of the adjuvant to produce severe arthritis, indicating that TLR9-mediated immune response determines the severity of autoimmune inflammation. Since mycobacterial DNA can be detected weeks after CFA inoculation, CpG DNA may augment autoimmune inflammation throughout the course of arthritis. These observations lend support to the notion that cathepsin K contributes to inflammation through TLR9 signaling.

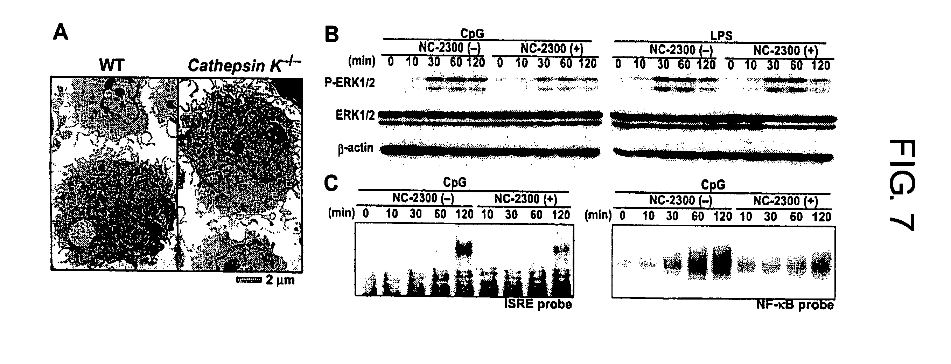

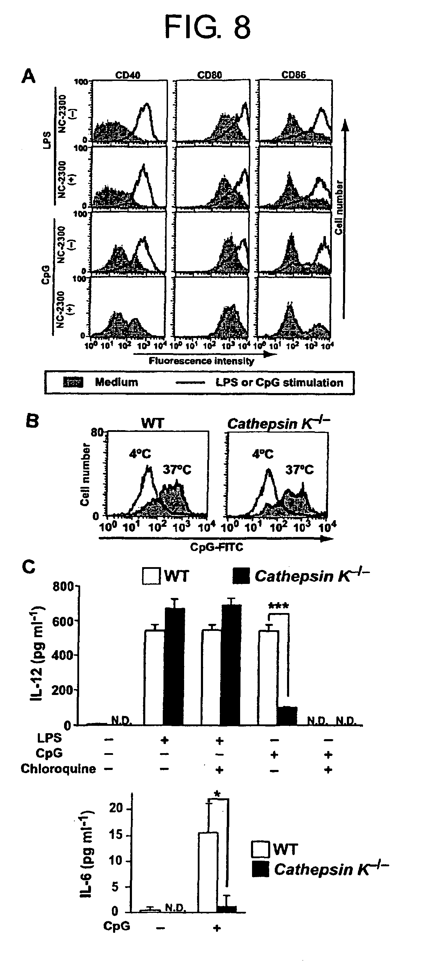

After being taken up into the cells, CpG DNA locates within the endosomal compartment, where CpG DNA binds to TLR9. The binding of CpG with TLR9 results in activation of MyD88 and downstream signaling such as the mitogen-activated protein kinase (MAPK), interferon regulatory factor (IRF) and nuclear factor-KB (NF-κB) pathways. Activation of these pathways leads to the production of inflammatory cytokines and upregulation of cell surface molecules such as CD40, CD80, and CD86 in DCs. NC-2300 had no effects on the in vitro development of BM-DCs, and transmission electron microscopy analysis revealed that BM-DCs derived from cathepsin K^{-/-} mice (mice having a defect of cathepsin K) exhibited a normal morphology (Fig. 7A). Next, the signaling events induced by CpG in DCs were tested. CpG-induced, but not LPS-induced, phosphorylation of extracellular-signal regulated kinases 1/2 (ERK 1/2) in DCs was suppressed by cathepsin K inactivation (Fig. 7B). Electrophoretic mobility shift assay (EMSA) revealed CpG-induced activation of IRF and NF-κB to be suppressed by cathepsin K inactivation in DCs (Fig. 7C). In addition, NC-2300 treatment reduced expression of CD40, CD80, and CD86 in DCs (Fig. 8A). Thus, cathepsin K inactivation leads to the blockade of essentially all the downstream pathways of TLR9 signaling in DCs, suggesting that cathepsin K plays a critical role in the signaling events proximal to TLR9. However, the endocytosis of CpG into DCs was not affected in cathepsin K^{-/-} DCs (Fig. 8B).

A marked decrease in the CpG induction of cytokines IL-12 and IL-6 was observed in cathepsin K^{-/-} DCs, whereas the induction of these cytokines in response to LPS was not affected (Fig. 8C). Chloroquine, an inhibitor of endosomal acidification, is known to inhibit TLR9 signaling. It is reported that TLR9 interacts with CpG more strongly under acidic pH conditions, but the detailed mechanism underlying the role of endosomal acidification in TLR9 signaling remains unclear. The selective inhibition of CpG-TLR9 signaling by cathepsin K ablation was similar to the effects of chloroquine, suggesting that chloroquine and cathepsin K inactivation may target the same signaling pathway. Cytokine induction in cathepsin K^{-/-} DCs was further suppressed by the addition of chloroquine (Fig. 8C), suggesting that other pH-sensitive enzymes also contribute albeit only minimally. Since cathepsins are known to be localized in the endosome and they are active exclusively under acidified conditions, it is inferred that the effects of chloroquine are mediated by the inhibition of these enzymes, mainly through cathepsin K inhibition. It remains to be elucidated how cathepsin K regulates CpG-TLR9 signaling in the endosome, but the results in pharmacological inhibition clearly indicates that the proteolytic activity of this enzyme is crucial for the mechanism. It is conceivable that cathepsin K may be involved in the degradation of proteins that inhibit the interaction between CpG and TLR9, or cathepsin K-mediated proteolysis may result in the conformational change of TLR9 which augments its interaction with CpG, although it cannot be ruled out the possibility that cathepsin K degrades an cytoplasmic protein that modifies the proximal TLR9 signaling.

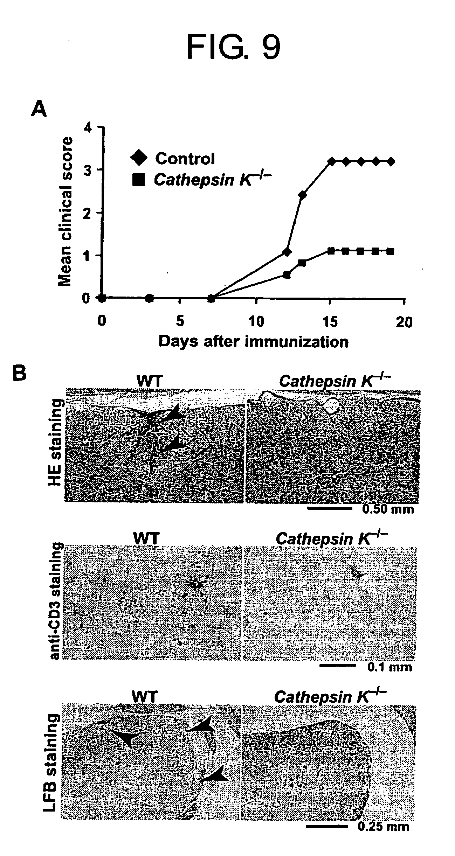

To clearly demonstrate that cathepsin K plays a critical role in autoimmune inflammation in an osteoclast-independent manner, cathepsin K^{-/-} mice was subjected to an experimental autoimmune encephalomyelitis (EAE) model, in which TLR9 signaling plays an important role. The frequency of the onset of EAE was not different between wild type (WT) and cathepsin K^{-/-} mice, but the severity of the paralytic symptoms was much lower in the cathepsin K^{-/-} mice than the WT mice (Fig. 9A). Histological analysis of spinal cords demonstrated dramatically decreased inflammation (Fig. 9B, upper, HE staining), T-cell infiltration (Fig. 9B, middle, anti-CD3 staining) and demyelination (Fig. 9B, lower, LFB staining) in cathepsin K^{-/-} mice.

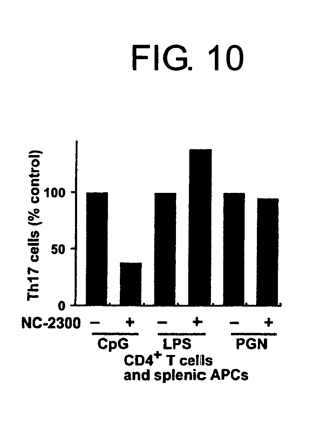

Since Th17 cells play an essential role in the autoimmune inflammation in EAE, we examined the effect of cathepsin K inactivation on the ability of DCs to induce Th17 cells. DCs were cultured with CD4⁺ T cells in the presence of CpG, LPS, or PGN, and the effect of NC-2300 on the induction of Th17 cells was determined by the intracellular staining of IL-17. Interestingly, the ability of DCs to induce Th17 cells was markedly inhibited by cathepsin K inactivation only when DCs were stimulated with CpG (Fig. 10).

Taken together with the results on the role of cathepsin K in CpG-induced cytokine expression in DCs, the impaired induction of Th17 cells in cathepsin K inactivation was caused, at least in part, by the reduced DC expression of cytokines which are involved in the induction and expansion of Th17 cells such as IL-6 and IL-23.

As is shown above, cathepsin K, which was thought to be an osteoclast-specific enzyme, plays a critical role in the immune system. Cathepsin K functions under the acidified conditions in the endosome, where engagement of CpG by TLR9 takes place, and plays an important role in the signaling events proximal to TLR9. It has been unclear how chloroquine, an inhibitor of endosome acidification, inhibits CpG-mediated TLR9 signaling, but the role of cathepsin K in TLR9 signaling fits very well with this observation. Chloroquine has long been used in the treatment of rheumatoid arthritis. The concept of chloroquine as a cathepsin K inhibitor may provide a molecular and cellular basis for its clinical efficacy. It has been recently reported that another antirheumatic drug, gold thiomalate, binds to the catalytic domain of cathepsin K. The direct substrate of cathepsin K remains to be elucidated, but pharmacological inhibition and gene disruption studies collectively suggest that cathepsin K plays an unexpected immunological role in DC-specific TLR9 signaling.

The importance of cathepsin K in the regulation of the immune system is underscored by the observations that a cathepsin K inhibitor suppressed autoimmune inflammation in an arthritis model and cathepsin K-deficient mice were resistant to EAE, the onset of which is clearly independent of osteoclasts. Although the detailed mechanisms remain to be identified, Th17 regulation is here shown to be one of the mechanisms underlying the cathepsin K regulation of autoimmune inflammation. Thus, careful attention should be paid to the side effects of cathepsin K inhibitors on the immune system in the treatment of osteoporosis, whereas they may have dual benefits in the treatment of autoimmune arthritis, the pathogenesis of which is dependent on both Th17 cells and osteoclasts.

As is described above, the cathepsin inhibitor, an active ingredient of the present invention, regulates TLR signaling, suppresses induction of Th17 cells, and inhibits induction of IL-6, IL-12, IL-17, or IL-23. Therefore, the cathepsin inhibitor can be used as an agent for treating diseases involved with those shown above. The cathepsin inhibitor can particularly be used as an agent for treating an autoimmune disease such as systemic lupus erythematosus, lupus nephritis, crohn's erythematosus, lupus nephritis, crohn's disease, psoriasis, acute disseminated encephalomyelitis, or multiple sclerosis, an infective disease, an immune disease such as asthma, a bone disease, or a cancer.

The cathepsin inhibitor is particularly in treating an autoimmune disease such as systemic lupus erythematosus, lupus nephritis, crohn's disease, psoriasis, or acute disseminated encephalomyelitis.

The cathepsin inhibitor, an active ingredient of the present invention, can be administered by either oral or parenteral route.

For instance, the compound can be processed to give pellets, granule, powder, capsule, suspension, injection, suppository, and the like.

For the preparation of these pharmaceuticals, ordinary additives such as vehicles, disintegrators, binders, lubricants, dyes, and diluents can be used. As the vehicles, lactose, D-mannitol, crystalline cellulose and glucose can be mentioned. Further, there can be mentioned starch and carboxymethylcellulose calcium (CMC-Ca) as the disintegrators, magnesium stearate and talc as the lubricants, and hydroxypropylcellulose (HPC), gelatin and polyvinylpyrrolidone (PVP) as the binders.

The compound of the invention can be administered to an adult generally in an amount of 0.1 mg to 100 mg a day by parenteral administration and 1 mg to 2,000 mg a day by oral administration. The dosage can be adjusted in consideration of age and conditions of the patient.

The invention is further described by the following non-limiting examples.

### Examples

Materials and methods used in the present invention are described below.

### Materials and Methods

### 1. Animals

We used two lines of *cathepsin* K-deficient mice; the *cathepsin* K-deficient mice described previously P. Saftig et al., Proc. Natl. Acad. Sci. USA95, 13453 (1998) and another *cathepsin* K-deficient strain (*cathepsin K*^{Cre/Cre}), which was generated by the intercrossing of cathepsin K-Cre knock-in mice (*cathepsin K*^{Cre/+}). All of the animals, including the BALB/c-background DO11.10 TCR-transgenic mice, which recognize the 323-339 peptide of ovalbumin in the context of I-Ad, were maintained in a specific pathogen-free environment, and all animal experiments were performed with the approval of the institutional committee of Tokyo Medical and Dental University.

### 2. Generation of dendritic cells (DCs) and macrophages from bone marrow (BM) cells

Immature DCs were generated from mouse BM, as described in K. Inaba et al., J. Exp. Med. 176,1693 (1992). In brief, BM cells were cultured with 10 ng ml⁻¹ murine GM-CSF or 10 ng ml⁻¹ murine Flt3L in RPMI 1640 medium supplemented with 10% FBS for 6 days. Loosely adherent cells were harvested by gentle pipetting and were used as BM-derived DCs. To obtain BM-derived macrophages, BM cells were cultured with 10 ng ml⁻¹ M-CSF in α-MEM medium supplemented with 10% FBS for 2 days, and adherent cells were used as BM-derived macrophages. For the pathogen-associated molecular pattern (PAMP) stimulation, cells were plated at 5 x 10⁵ cells ml⁻¹ in 24-well plates, and cultured with 10 ng ml⁻¹ Lipopolysaccharide (LPS), 30 µg ml⁻¹ Peptidoglycan Type I (PGN), or Endotoxin-free phosphorothioate-stabilized CpG oligodeoxynucleotides (CpG ODN).

### 3. Enzyme-linked immunosorbent assay (ELISA)

Cells were cultured for 3-24 hours with or without PAMPs or plate-bound anti-CD3/CD28 antibodies in the presence or absence of NC-2300, and concentration of cytokines in the culture supernatant was determined by ELISA kits, according to the manufacturer's instructions.

### 4. Cell proliferation assay

Cell proliferation was evaluated by using a Cell Proliferation ELISA kit according to the manufacturer's protocol.

### 5. Flowcytometry

Before staining, cells were incubated with mouse BD Fc Block to minimize nonspecific staining. The cells were then stained with fluorophore-conjugated monoclonal Abs. The following fluorophore-conjugated mAbs were used; Allophycocyanin (APC)-conjugated anti-CD3e (145-2C11), fluorescein isothiocyanate (FITC)-conjugated anti-CD4 (RM4-5), CD11c (HL3), CD40 (3/23), CD80 (16-10A1), CD86 (GL1), I-A^{d} (AMS-32.1), and Phycoerythrin (PE)-conjugated anti-mouse IL-17 (TC11-18H10). For intracellular cytokine staining, cells were collected and left unstimulated or were stimulated for 5 hours with PMA (40 ng ml⁻¹) and ion-omycin (4 µg ml⁻¹) in the presence of GolgiPlug at the recommended concentration. Standard intracellular cytokine staining was performed as follows; cells were first stained extracellularly with APC-conjugated anti-CD3 and FITC-conjugated anti-CD4 mAbs, then were fixed and permeabilized with Cytofix/Cytoperm solution, and finally were stained intracellularly with PE-conjugated anti-IL-17 mAb. Flowcytometric analysis was performed by FACScan with CellQuest software or FACSCanto II with Diva software.

### 6. Th17 cell differentiation

CD4-positive T cells and splenic DCs were isolated from the spleen using a magnetic sorter and anti-CD4 microbeads. These cells were stimulated with plate-bound anti-CD3 and anti-CD28 mAbs (1 µg ml⁻¹ each) for 3 days in the presence of 2 ng ml⁻¹ human TGF-β, 10 µg ml⁻¹ each of anti-IFN-y and anti-IL-4 mAbs, with or without CpG ODN, LPS or PGN.

### 7. Electron Microscopy (EM)

DCs were fixed in 2.5% EM-grade glutaraldehyde in 100 mM phosphate buffer (pH 7.4) for 2 hours, then washed with 100 mM phosphate buffer (pH 7.4) for 2 hours, post-fixed with 1% osmium tetroxide in 100 mM phosphate buffer (pH.7.4) for 2 hours, and processed for the EM specimen.

### 8. Endocytosis assay

FITC-conjugated-ovalbumin (ovalbumin-FITC) or FITC-conjugated CpG ODN (CpG-FITC) was added to the cell cultures at the final concentration of 100 µg ml⁻¹ or 1 µM, respectively. Then the cells were incubated for 30 min at 4°C or 37°C in a 5% CO₂ humidified environment. The cells were then washed three times with cold PBS containing 1% FCS and 0.01% NaN₃ and were analyzed by flowcytometry. Trypan blue was used for quenching extracellular fluorescence.

### 9. RT-PCR analysis

RT-PCR analysis was performed as described in K. Sato et al., Nat Med 12, 1410 (2006). The following PCR primers were used.

Cathepsin K:
5'-ATACGTTACTCCAGTCAAGAACCAG-3' (sense) and
   5'-ATAATTCTCAGTCACACAGTCCACA-3' (antisense);
(β-actin:
5'-GTACGACCAGAGGCATACAGG-3' (sense) and
   5'-GATGACGATATCGCTGCGCTG-3' (antisense).

### 10. Quantitative RT-PCR

Quantitative RT-PCR was performed as described in K. Sato et al., Nat Med 12, 1410 (2006). The following primers were used.

I123a:
5'-ATGCTGGATTGCAGAGCAGTA-3' (sense) and
5'-ACGGGGCACATTATTTTTAGTCT-3' (antisense);
I112a:
5'-CCCTTGCCCTCCTAAACCAC-3' (sense) and 5'-AAGGAACCCTTAGAGTGCTTACT-3' (antisense);
I112b:
5'-GACACGCCTGAAGAAGATGAC-3' (sense) and 5'-TAGTCCCTTTGGTCCAGTGTG-3' (antisense);
Ifnb1:
5'-CAGGCAACCTTTAAGCATCAG-3' (sense) and 5'-CCTTTGACCTTTCAAATGCAG-3' (antisense).

The level of mRNA expression was normalized with that of β-actin expression.

### 11. Immunoblot analysis

DCs were cultured in low serum medium (0.1 % FBS) for 4 hours to reduce background and then stimulated with CpG ODN or LPS in the presence or absence of NC-2300. The stimulated cells were subjected to immunoblot analyses as described in K. Sato et al., Nat Med12, 1410 (2006), using Abs for ERK-1/2, phosphorylated ERK-1/2 and β-actin.

### 12. Electrophoretic mobility shift assay (EMSA)

DCs were cultured in low serum medium (0.1 % FBS) for 4 hours to reduce background and then stimulated with CpG ODN or LPS in the presence or absence of NC-2300. EMSA was performed as described in H. Hemmi et al., Nature 408, 740 (2000). The oligonucleotide probes used for EMSA were as follow.

NF-κB:
5'-ATCAGGGACTTTCCGCTGGGGACTTTCC-3' (sense) and
5'-GGAAAGTCCCCAGCGGAAAGTCCCTGAT-3' (antisense);
ISRE:
5'-GATCCATGCCTCGGGAAAGGGAAACCGAAACTGAAGCC-3' (sense) and
5'-GGCTTCAGTTTCGGTTTCCCTTTCCCGAGGCATGGATC-3' (antisense).

Oligonucleotide probes were end-labeled with [γ-³²P] ATP by using T4 polynucleotide kinase.

### 13. Experimental autoimmune encephalomyelitis (EAE)

The murine MOG₃₅₋₅₅ peptide (MEVGWYRSPFSRVVHLYRNGK) was synthesized at Division of Molecular Biology, Institute of Medical Science, University of Tokyo. Six- to 12-week-old mice were immunized with the MOG peptide (250 µg/mouse) emulsified in CFA containing 250 µg ml⁻¹ *Mycobacterium tuberculosis* H37RA (day 0). After immunization, the mice were intraperitoneally injected with pertussis toxin (200 ng/mouse) at day 0 and 2. Animals were scored for clinical signs of EAE for 3 weeks using the following criteria: 0, no clinical signs; 1, limp tail (tail paralysis); 2, complete loss of tail tonicity or abnormal gait; 3, partial hind limb paralysis; 4, complete hind limb paralysis; 5, forelimb paralysis or moribund; and 6, death. Histological analysis was performed as follows. Mice were perfused with PBS followed by 4% paraformaldehyde. Spinal cords were embedded in paraffin and stained with hematoxylin and eosin to assess inflammation, with luxol fast blue to assess the degree of demyelination, or immunostained for CD3 with anti-CD3e Ab. to assess T-cell infiltration.

### 14. Statistical analysis

Statistical analysis was performed using Student's *t*-test, Fisher's exact test, and analysis of variance followed by Tukey-Kramer test or Dunnett's multiple comparison test when applicable. All the statistically analyzed data are expressed as mean ± s.e.

### [Example 1]

Comparison of effects of NC-2300 and alendronate on adjuvant arthritis

### (1) Radiological (soft X-rays) assessment of ankle joints

NC-2300 was orally administered to a rat model of adjuvant arthritis, and was compared with alendronate in pharmacological test. Rats were injected with complete Freund's adjuvant on day 0. NC-2300, alendronate or vehicle was administered to the rats at the indicated doses daily starting on day 0 up to day 35.

As the result of radiological (soft X-rays) assessment of ankle joints (day 36), severe joint destruction (joint space narrowing, erosion, and bone spur formation) was observed in the vehicle- or alendronate-treated rats but not in the NC-2300-treated rats (Fig. 2, A and B).

### (2) Effect of NC-2300 or alendronate on periarticular osteoporosis

The proximal tibia was radiologically examined (using soft X-rays) on day 36 to measure the effect of NC-2300 or alendronate on periarticular osteoporosis to measure effect of NC-2300 or alendronate on periarticular osteoporosis. Bone mineral density of the proximal tibia assessed by pQCT (day 36).

Bone mineral density (BMD) showed that both compounds had a comparable inhibitory effect on periarticular osteoporosis in the proximal tibia (Fig. 2, C and D).

### (3) Effect of NC-2300 or alendronate on paw swelling and evaluation of adjuvant arthritis in hind paw administered with NC-2300 or vehicle

Assessment for hind paws of adjuvant arthritis rats treated with NC-2300 or vehicle. The results are set forth in Fig. 3A.

Adjuvant arthritis in hind paw administered with NC-2300 or vehicle was evaluated. The results are set forth in Fig. 3B.

NC-2300 ameliorated paw swelling in the course of arthritis without affecting the onset rate of arthritis (Fig. 3, A and B).

### (4) Recovery of locomotion activity of arthritic rats by NC-2300

Recovery of locomotion activity of arthritic rats by NC-2300 (day 35) was measured. The results are set forth in Fig. 3C.

High dose treatment of NC-2300 significantly ameliorated the arthritis-induced incapacitation of rearing behavior (left) and reduced the severity of the hind limb lameness (right). (Fig. 2B, D, and Fig. 3A and C: *n* = 10, **P* < 0.05, ***P* < 0.01, ****P* < 0.001 (versus vehicle-treated group). N.D., not detected.

### [Example 2]

Selective suppression of Toll-like receptor 9 (TLR9) response in bone marrow-derived dendritic cells (BM-DCs) by cathepsin K inhibition

BM-DCs were induced by granulocyte-macrophage colony-stimulating factor (GM-CSF), unless otherwise indicated.

### (1) RT-PCR analysis of the mRNA expression of cathepsin K in BM-DCs

We analyzed the expression of cathepsin K in T cells, macrophages and DCs, and examined the effects of a cathepsin K inhibitor on the activation of these cells.

RT-PCR analysis of the mRNA expression of cathepsin K in BM-DCs is set forth in Fig. 4A.

Cathepsin K mRNA was barely detected in non-adherent BM cells or splenic T cells by reverse transcriptase-polymerase chain reaction (RT-PCR) analysis (Fig. 4A). NC-2300 had no effect on the proliferation or IL-2 production of T cells stimulated with anti-CD3/CD28 antibodies.

NC-2300 had no significant effects on the activation of BM-derived macrophages stimulated by PAMPs. BM-derived DCs were revealed to express a detectable level of cathepsin K mRNA (Fig. 4A).

### (2) Enzymatic activity of cathepsin K in BM-DCs

Enzymatic activity of cathepsin K in BM-DCs was measured.

BM-DCs were pre-treated with or without the cathepsin K inhibitor NC-2300 for 24 hours and washed extensively with cold phosphate-buffered saline (PBS), and cell lysates were subjected to enzymatic assay. Cathepsin K activity was measured with cathepsin K specific substrate, Z-Gly-Pro-Arg-MCA (GPR).

Although cathepsin K mRNA expression in DCs was much lower than that in osteoclasts, cathepsin K activity in DCs was confirmed using a fluorogenic cathepsin K-specific substrate, Z-Gly-Pro-Arg-MCA (Z, benzyloxycarbonyl; MCA, 4-methylcoumary-7-amide), and the activity was inhibited by NC-2300 in a dose-dependent manner (Fig. 4B) .

### (3) Effects of cathepsin K inhibition on TLR-stimulated cytokine production

Effects of cathepsin K inhibition on TLR-stimulated cytokine production were measured.

BM-DCs were stimulated with peptidoglycan (PGN), lipopolysaccharide (LPS), and unmethylated CpG oligodeoxynucleotides (CpG) for 24 hours and the culture supernatants were subjected to ELISA. **P* < 0.05, ****P* < 0.001 (versus control).

Enzyme-linked immunosorbent assay (ELISA) revealed that the production of cytokines such as IL-12 and IL-23 by DCs was significantly inhibited by NC-2300 when they were stimulated with oligodeoxynucleotides containing unmethylated CpG motif (CpG: the TLR9 ligand), but not with peptidoglycan (PGN: the TLR2 ligand) or lipopolysaccharide (LPS: the TLR4 ligand) (Fig. 5A).

### (4) Effect of NC-2300 on cytokine mRNA expression in CpG-treated BM-DCs.

Effect of NC-2300 on cytokine mRNA expression in CpG-treated BM-DCs was measured.

BM-DCs were generated by either GM-CSF or Flt3L. Total RNA was extracted 6 hours after CpG stimulation.

As the result, CpG-induced expression of IL-6, IL-12 and IL-23 in BM-DCs was downregulated at the mRNA level (Fig. 5B), and IFN production by Flt3L-induced DCs in response to CpG was suppressed by NC-2300.

### [Example 3]

Effect of NC-2300 on cytokine mRNA expression in poly(I:C) (TLR3 ligand) and R837 (TLR7, 8 ligand)-treated DCs derived from mouse spleen.

Effect of NC-2300 on cytokine mRNA expression in poly(I:C) (TLR3 ligand) and R837 (TLR7, 8 ligand)-treated DCs derived from mouse spleen was measured.

Spleens were excised from male mice of 8 to 10 weeks. Dendritic cells (DCs) were separated from the spleens using pan DC microbeads (Miltenyi Biotec, Bergisch Gladbach, Germany), according to the manufacturer's instructions.

Total RNA was extracted 3 hours after stimulation.

As the result, cytokine mRNA expression in poly(I:C) and R837-treated DCs derived from mouse spleen was suppressed by NC-2300 at mRNA level (Fig. 6).

A cathepsin inhibitor, E64d also suppressed cytokine mRNA expression in poly(I:C) and R837-treated DCs derived from mouse spleen at mRNA level.

### [Example 4]

Cathepsin K regulation of TLR9-mediated immune responses and autoimmune inflammation

### (1) Electron micrographs of WT and cathepsin K^{-/-} bone marrow-derived dendritic cells (DCs).

Electron micrographs of wild type (WT) and *cathepsin K*^{-/-} (mice having a defect of cathepsin K gene) bone marrow-derived dendritic cells (DCs) are shown in Fig. 7A.

NC-2300 had no effects on the *in vitro* development of BM-DCs, and transmission electron microscopy analysis revealed that BM-DCs derived from *cathepsin K*^{*-*/}*⁻* mice exhibited a normal morphology (Fig. 7A).

### (2) Suppression of ERK phosphorylation by NC-2300 in CpG-stimulated DCs.

We next tested the signaling events induced by CpG in DCs. CpG-induced, but not LPS-induced, phosphorylation of extracellular-signal regulated kinases 1/2 (ERK 1/2) in DCs was suppressed by cathepsin K inactivation (Fig. 7B) .

### (3) Effect of cathepsin K inhibition on CpG-induced activation of transcription factors, interferon regulatory factor and NF-κB (EMSA)

Effect of cathepsin K inhibition on CpG-induced activation of transcription factors, interferon regulatory factor (Fig. 7C, left) and NF-κB (Fig. 7C, right) was measured.

Electrophoretic mobility shift assay (EMSA) revealed CpG-induced activation of IRF and NF-κB to be suppressed by cathepsin K inactivation in DCs (Fig. 7C).

### (4) Impaired TLR9-mediated expression of cell surface molecules CD40, CD80 and CD86 in NC-2300-treated DCs

Impaired TLR9-mediated expression of cell surface molecules CD40, CD80 and CD86 in NC-2300-treated DCs was measured.

DCs were stimulated with CpG or LPS for 48 hours and analyzed by flowcytometry.

As the result, NC-2300 treatment reduced expression of CD40, CD80 and CD86 in DCs (Fig. 8A). Thus, cathepsin K inactivation leads to the blockade of essentially all the downstream pathways of TLR9 signaling in DCs, suggesting that cathepsin K plays a critical role in the signaling events proximal to TLR9.

### (5) Normal uptake of CpG in cathepsin K^{-/-} DCs

Normal uptake of CpG in *cathepsin K*^{-/-} DCs was measured. DCs were incubated with CpG-FITC at 4°C or 37°C, and endocytic activity was examined by flowcytometry.

As the result, the endocytosis of CpG into DCs was not affected in *cathepsin K*^{*-*/}*⁻* DCs (Fig. 8B).

### (6) Cytokine production in WT or cathepsin K^{-/-} DCs in response to LPS or CpG

Cytokine production in WT or *cathepsin K*^{-/-} DCs in response to LPS or CpG was measured. Chloroquine was added 1 hour before the stimulation to suppress endosomal acidification. N.D., not detected. **P* < 0.05, ****P* < 0.001 (versus WT).

As the result, we observed a marked decrease in the CpG induction of cytokines IL-12 and IL-6 in *cathepsin K*^{*-*/-} DCs, whereas the induction of these cytokines in response to LPS was not affected (Fig. 8C).

Cytokine induction in *cathepsin K*^{-/-} DCs was further suppressed by the addition of chloroquine (Fig. 8C), suggesting that other pH-sensitive enzymes also contribute albeit only minimally.

We inferred that the effects of chloroquine are mediated by the inhibition of these enzymes, mainly through cathepsin K inhibition.

It is apparent from the cathepsin K inhibition effects of the agents that proteinase activities of the enzymes play an important role in CpG-TLR9 signaling.

### (7) Severity of experimental autoimmune encephalomyelitis (EAE) in cathepsin K^{-/-} mice

To clearly demonstrate that cathepsin K plays a critical role in autoimmune inflammation in an osteoclast-independent manner, we subjected *cathepsin K*^{-/-} mice to an experimental autoimmune encephalomyelitis (EAE) model, in which TLR9 signaling plays an important role.
Fig. 9A shows EAE clinical scores in control (WT and *ca thepsin K*^{*+*/}*⁻)* and *ca thepsin K*^{-/-} mice.
Fig. 9B shows histological analysis of spinal cord sections from representative control and *cathepsin K*^{*-*/*-*} mice at day 21 after immunization. Lumbar spinal cord sections from WT and *cathepsin K*^{-/-} mice was stained with hematoxylin and eosin (HE staining) to assess inflammation, immunostained for CD3 (anti-CD3 staining), and stained with luxol fast blue (LFB staining) to assess myelin content. Arrowheads indicate inflammatory cellular infiltrates (HE staining) and demyelinated areas (LFB staining).

As the result, the frequency of the onset of EAE was not different between wild-type (WT) and *cathepsin K*^{*-*/*-*} mice, but the severity of the paralytic symptoms was much lower in the *cathepsin K*^{-/-} mice than the WT (control) mice (Fig. 9A). Histological analysis of spinal cords demonstrated dramatically decreased inflammation (Fig. 9A, upper, HE staining), T-cell infiltration (Fig. 9A, middle, anti-CD3 staining) and demyelination (Fig. 9B, lower, LFB staining) in *ca thepsin K*^{-/-} mice.

### (8) Cathepsin K inactivation results in defective Th17 polarization in response to CpG, but not LPS or PGN

Cathepsin K inactivation results in defective Th17 polarization in response to CpG, but not LPS or PGN were measured. CD4⁺ T cells and DCs were sorted from the spleen, and cocultured in the presence of CpG, LPS, or PGN for 3 days. After the culture, percentages of CD3e⁺CD4⁺IL-17⁺ cells were analyzed by flowcytometry.

The effect of NC-2300 on the induction of Th17 cells was determined by the intracellular staining of IL-17. As the result, the ability of DCs to induce Th17 cells was markedly inhibited by cathepsin K inactivation only when DCs were stimulated with CpG (Fig. 10).

### Brief description of the drawings

### [Figure 1]

Structural formula of NC-2300

### [Figure 2]

Figure showing experimental results of adjuvant arthritis treated with NC-2300 or alendronate

### [Figure 3]

Figure showing experimental results of adjuvant arthritis treated with NC-2300 or alendronate

### [Figure 4]

Figure showing cathepsin K mRNA expression and cathepsin K activity

### [Figure 5]

Figure showing selective inhibition of TLR-9 signaling of bone marrow derived dendritic cells (BM-DCs) by cathepsin inhibition

### [Figure 6]

Figure showing selective inhibition of TLR-3, 7, 8 signaling by cathepsin inhibition

### [Figure 7]

Electron micrograph and figure showing effect of cathepsin inhibition

### [Figure 8]

Figure showing TLR9-mediated immune reaction and autoimmune inflammation, and regulation by cathepsins

### [Figure 9]

Figure showing EAE clinical scores in wild-type mice and cathepsin K^{-/-} mice.

### [Figure 10]

Figure showing effect of NC-2300 on induction of Th17 cells

### Figure legends

Fig. 2A
   Soft X-rays assessment of ankle joints
Fig. 2B
   Soft X-rays assessment of ankle joints
Fig. 2C
   Measurement of bone density
Fig. 2D
   Bone mineral density analysis
Fig. 3A
   Effect on paw swelling
Fig. 3B
   Assessment for hind paws
Fig. 3C
   Recovery of locomotion activity
Fig. 4A
   RT-PCR analysis of mRNA expression
Fig. 4B
   Measurement of cathepsin K activity
Fig. 5A
   Effect of cathepsin K inhibition
Fig. 5B
   Cytokine mRNA expression
Fig. 6A
   Suppression of TLR3 signaling
Fig. 6B
   Suppression of TLR7, 8 signaling
Fig. 7A
   Electron micrograph
Fig. 7B
   Phosphorylation of ERK 1/2
Fig. 7C
   Effect of cathepsin K inactivation
Fig. 8A
   Flowcytometry analysis
Fig. 8B
   Flowcytometry analysis
Fig. 9A
   EAE clinical score
Fig. 9B
   Histological analysis of spinal cords

## Claims

1. A modulator for signaling of TLR which contains a cathepsin inhibitor as an active ingredient.

2. A therapeutic agent for treating diseases associated with signaling of TLR which contains a cathepsin inhibitor as an active ingredient.

3. The modulator or the therapeutic agent defined in claim 1 or 2, wherein the TLR is selected from the group consisting of TLR3, TLR7, TLR8, and TLR9.

4. The modulator or the therapeutic agent defined in claim 1 or 2, wherein the TLR is TLR9.

5. A therapeutic agent for treating diseases associated with induction of Th17 cells which contains a cathepsin inhibitor as an active ingredient.

6. A therapeutic agent for treating diseases associated with production of IL-6, IL-12, IL-17, or IL-23 which contains a cathepsin inhibitor as an active ingredient.

7. The therapeutic agent for treating diseases defined in one of claims 2 to 6, wherein the disease is selected from the group consisting of immune disease, bone disease, and cancer disease.

8. The therapeutic agent for treating diseases defined in one of claims 2 to 6, wherein the disease is autoimmune disease.

9. A therapeutic agent for treating systemic lupus erythematosus, lupus nephritis, crohn's disease, psoriasis, or acute disseminated encephalomyelitis which contains a cathepsin inhibitor as an active ingredient.

10. The modulator or the therapeutic agent for treating diseases defined in one of claims 1 to 9, wherein the cathepsin inhibitor is selected from the group consisting of cathepsin K inhibitor, cathepsin L inhibitor, cathepsin B inhibitor, cathepsin H inhibitor, and cathepsin S inhibitor.

11. The modulator or the therapeutic agent for treating diseases defined in one of claims 1 to 9, wherein the cathepsin inhibitor is cathepsin K inhibitor.

12. The modulator or the therapeutic agent for treating diseases defined in one of claims 1 to 9, wherein the cathepsin inhibitor is selected from the group consisting of N-[1-[(cyanomethyl)carbamoyl]cyclohexyl]-4-(4-propylpiperazin-1-yl)benzamide, N-[(1S)-3-methyl-1-[[(4S,7R)-7-methyl-3-oxo-1-(pyridin-2-ylsulfonyl)hexahydro-1H-azepin-4-yl]carbamoyl]butyl]-1-benzofuran-2-carboxamide, (2R)-N-cyanomethyl-4-methyl-2-(4'-piperazin-1-yl-1,1'-biphenyl-3-yl)pentanamide, N-[3-[(2Z)-2-(3-methyl-1,3-thiazolidin-2-ylidene)hydrazino]-2,3-dioxo-1-tetrahydro-2H-pyran-4-ylpropyl]cycloheptanecarboxamide, N-cyanomethyl-4-methyl-2-[2,2,2-trifluoro-1-(4'-methylsulfonyl-1,1'-biphenyl-4-yl)ethylamino]pentanamide, and monosodium (2S,3S)-3-[[(1S)-1-isobutoxymethyl-3-methylbutyl]carbamoyl]oxirane-2-carboxylate.

13. The modulator or the therapeutic agent for treating diseases defined in one of claims 1 to 9, wherein the cathepsin inhibitor is an epoxysuccinamide derivative represented by the formula (1) or a physiologically acceptable salt thereof: 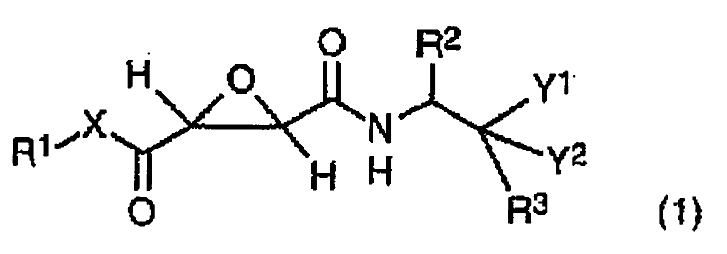 wherein
R¹ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
R² represents an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
R³ represents a hydrogen atom, an alkyl group having 1 to 10 carbon atoms, an alkenyl group having 2 to 10 carbon atoms, an alkynyl group having 2 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
X represents -O- or -NR⁴- in which R⁴ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
Y¹ represents OR⁵ in which R⁵ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, SR⁶ in which R⁶ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, or NR⁷R⁸ in which R⁷ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, an acyl group having 2 to 20 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, and R⁸ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
Y² represents a hydrogen atom or an alkyl group having 1 to 10 carbon atoms; or
Y¹ and Y² in combination with each other can form =O, =S, =N-R⁹ in which R⁹ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms, or =N-OR¹⁰ in which R¹⁰ represents a hydrogen atom, an alkyl group having 1 to 10 carbon atoms, an aryl group having 6 to 20 carbon atoms, an aralkyl group comprising an aryl group having 6 to 20 carbon atoms and an alkyl group having 1 to 6 carbon atoms, a heterocyclic group having 3 to 12 carbon atoms, or a heterocyclic-alkyl group comprising a heterocyclic group having 3 to 12 carbon atoms and an alkyl group having 1 to 6 carbon atoms;
provided that each of the alkyl groups for R⁵ to R¹⁰ can have one or more substituents selected from the group consisting of hydroxyl, amino, an alkylamino group having 1 to 6 carbon atoms, a dialkylamino group having 2 to 12 carbon atoms in total, an alkoxy group having 1 to 6 carbon atoms, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, and guanidino; and
each of the aryl groups and the heterocyclic groups for R¹ to R¹⁰ can have one or more substituents selected from the group consisting of an alkyl group having 1 to 6 carbon atoms, hydroxyl, amino, an alkylamino group having 1 to 6 carbon atoms, a dialkylamino group having 2 to 12 carbon atoms in total, an alkoxy group having 1 to 6 carbon atoms, a halogen atom, a haloalkyl group having 1 to 6 carbon atoms, cyano, nitro, carboxyl, an alkoxycarbonyl group having 2 to 7 carbon atoms, carbamoyl, an alkylaminocarbonyl group having 2 to 7 carbon atoms, a dialkylaminocarbonyl group having 3 to 13 carbon atoms in total, amidino, and guanidino.

14. The modulator or the therapeutic agent for treating diseases defined in claim 13, wherein R¹ in the formula (1) is a hydrogen atom or an alkyl group having 1 to 6 carbon atoms.

15. The modulator or the therapeutic agent for treating diseases defined in claim 13 or 14, wherein R² in the formula (1) is an alkyl group having 1 to 6 carbon atoms, phenyl, or benzyl.

16. The modulator or the therapeutic agent for treating diseases defined in one of claims 13 to 15, wherein R³ in the formula (1) is a hydrogen atom or an aryl group having 6 to 20 carbon atoms.

17. The modulator or the therapeutic agent for treating diseases defined in one of claims 13 to 16, wherein X in the formula (1) is -O-.

18. The modulator or the therapeutic agent for treating diseases defined in one of claims 13 to 17, wherein Y¹ in the formula (1) is hydroxyl, an alkoxy group having 1 to 6 carbon atoms, acetoxy, or an aralkyloxy group comprising an aryl group having 6 to 20 carbon atoms and an alkyl group having 1 to 6 carbon atoms.

19. The modulator or the therapeutic agent for treating diseases defined in one of claims 13, 14, and 17, wherein R² in the formula (1) is isobutyl or isopropyl, R³ is a hydrogen atom, Y¹ is OR⁵ in which R⁵ is defined in claim 13, and Y² is a hydrogen atom.

20. The modulator or the therapeutic agent for treating diseases defined in one of claims 13, 14, and 17, wherein R² in the formula (1) is isobutyl or isopropyl, R³ is an aryl group having 6 to 20 carbon atoms, Y¹ is OR⁵ in which R⁵ is defined in claim 13, and Y² is a hydrogen atom.

21. The modulator or the therapeutic agent for treating diseases defined in one of claims 13 to 17, wherein Y¹ and Y² in the formula (1) in combination with each other form =O.

22. The modulator or the therapeutic agent for treating diseases defined in one of claims 13 to 21, wherein the physiologically acceptable salt is an alkali metal salt.

23. The modulator or the therapeutic agent for treating diseases defined in one of claims 1 to 9, wherein the cathepsin inhibitor is monosodium (2S,3S)-3-[[(1S)-1-isobutoxymethyl-3-methylbutyl]carbamoyl]oxirane-2-carboxylate.
